(19) Europäisches Patentamt
European Patent Office
Office européen des brevets

(11) Publication number : **0 647 634 A1**

(12) # EUROPEAN PATENT APPLICATION

(21) Application number : **94202887.9**

(51) Int. Cl.[6] : **C07D 263/24,** C07D 263/16

(22) Date of filing : **05.10.94**

(30) Priority : **08.10.93 EP 93202856**

(43) Date of publication of application : **12.04.95 Bulletin 95/15**

(84) Designated Contracting States : **AT BE CH DE DK ES FR GB GR IE IT LI LU NL PT SE**

(71) Applicant : **DUPHAR INTERNATIONAL RESEARCH B.V**
**C.J. van Houtenlaan 36**
**NL-1381 CP Weesp (NL)**

(72) Inventor : **Brussee, Johannes**
**c/o Octrooibureau Zoan B.V.**
**P.O. Box 140**
**NL-1380 AC Weesp (NL)**
Inventor : **van der Gen, Arne**
**c/o Octrooibureau Zoan B.V.**
**P.O. Box 140**
**NL-1380 AC Weesp (NL)**
Inventor : **Warmerdam, Erwin G.J.C.**
**c/o Octrooibureau Zoan B.V.**
**P.O. Box 140**
**NL-1380 AC Weesp (NL)**
Inventor : **Kruse, Chris G.**
**c/o Octrooibureau Zoan B.V.**
**P.O. Box 140**
**NL-1380 AC Weesp (NL)**

(74) Representative : **Swaters, Pieter D. et al**
**OCTROOIBUREAU ZOAN B.V.**
**P.O. Box 140**
**NL-1380 AC Weesp (NL)**

(54) **Method of preparing a heterocyclic intermediate for the production of optically active aryloxy-substituted vicinal aminoalcohols.**

(57) The invention relates to a method of preparing a heterocyclic intermediate of the general formula

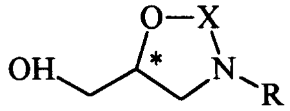

(I)

wherein

X is a carbonyl group, a thiocarbonyl group, a ($C_1$-$C_{10}$)(ar)alkylidene group or a dihydrocarbylsilyl group,

R is a straight or branched ($C_1$-$C_{10}$)alkyl group, optionally substituted with halogen, hydroxy, ($C_1$-$C_4$) alkoxy or protected hydroxy, or a phenyl($C_1$-$C_3$)alkyl or heteroaryl($C_1$-$C_3$)alkyl group, which groups are optionally substituted with 1-3 substituents, selected from the group consisting of hydroxy, ($C_5$-$C_{12}$)cycloalkyl, amino, nitro, halogen, cyano, alkoxy, alkylcarbonyloxy, alkylcarbonylamino, alkylsulphonylamino, alkylsulphonyl, alkylcarbonyl, and alkyl, wherein the alkyl groups have 1-5 carbon atoms, and which intermediate has either the R or the S configuration, by subjecting an optically active cyanohydrin of the general formula

EP 0 647 634 A1

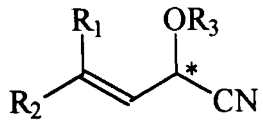

(II)

to a reduction-transimination-reduction sequence, using R - $NH_2$ as the primary amine, followed by a cyclization reaction and, finally, by an ozonolysis-reduction sequence.

The invention relates to a method of preparing a heterocyclic intermediate for the production of optically active aryloxy-substituted vicinal aminoalcohols. The invention also relates to an optically active heterocyclic compound, as well as to a method of its preparation and to its use.

A large number of pharmaceutically active agents belong to the class of aryloxy-substituted vicinal aminoalcohols, for example, the β-blockers (β-adrenoceptor antagonists) acebutolol, atenolol, metoprolol, betaxolol, bisoprolol, esmolol, metipranolol, practolol, alprenolol, oxprenolol, befunolol, propranolol, pindolol, carazolol, indenolol, mepindolol, moprolol, toliprolol, bucumolol, bufetolol, bunitrolol, bupranolol, butofilolol, carteolol, celiprolol, cetamolol, cloranolol, levobunolol, nadolol, penbutolol, talinolol, tertatolol, xibenolol and penirolol. All these pharmaceutically active agents contain a chiral centre in their molecular structure and therefore give rise to optical isomerism. It is generally known in the art that only one of the enantiomers displays the desired biological activity (the so-called eutomer) and that the presence of its optical antipode may lead to side effects. Therefore, it is generally deemed more and more desirable to administer the biologically active agent in the form of its pure eutomer rather than the racemic mixture.

A well-known method for the preparation of aryloxy-substituted vicinal aminoalcohols is the coupling of 1-(ar)alkylamino-2,3-propanediols or protected forms thereof with aromatic alcohols. The synthesis of optically active 1-alkylamino-2,3-propanediols or protected forms thereof has been described in literature, e.g. by Bianchi et al. in J. Org. Chem. 1988, 53, 5531-34. Bianchi et al. disclose the lipase-catalyzed stereoselective esterification of racemic alcohols, e.g. of 5-hydroxymethyl-3-alkyl-oxazolidin-2-one, as a method for the resolution of the racemic mixtures. In this manner the desired R or S isomers can be obtained in high enantiomeric purities. Although the yields are satisfactory, viz. approx. 40% calculated on starting racemate, it will be obvious that a "loss" of approx. 60%, mainly consisting of the undesired R-enantiomer of the above hydroxymethyl-oxazolidinone compound, is a serious drawback. Margolin (Enzyme Microb. Technol. 1993, Vol. 15, 266-268) has expressed this commonly felt disadvantage in a slightly different way, by stating: "However, none of the procedures proposed so far addresses an important issue of recovering an unwanted (R)-enantiomer. Unless this recovery is achieved, the low overall yield of the process will be a stumbling block for industrial application of these methods".

It is the objective of the present invention to provide an economically more attractive method of preparing an intermediate for the production of optically active aryloxy-substituted vicinal aminoalcohols.

This objective can be achieved by a method as described in the opening paragraph, which method is characterized according to the present invention in that a heterocyclic intermediate of the general formula

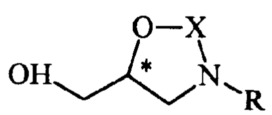

(I)

wherein

X is a carbonyl group, a thiocarbonyl group, a $(C_1-C_{10})$ (ar)alkylidene group or a dihydrocarbylsilyl group,

R is a straight or branched $(C_1-C_{10})$alkyl group, optionally substituted with halogen, hydroxy, $(C_1-C_4)$alkoxy or protected hydroxy, or a phenyl$(C_1-C_3)$alkyl or heteroaryl$(C_1-C_3)$alkyl group, which groups are optionally substituted with 1-3 substituents, selected from the group consisting of hydroxy, $(C_5-C_{12})$cycloalkyl, amino, nitro, halogen, cyano, alkoxy, alkylcarbonyloxy, alkylcarbonylamino, alkylsulphonylamino, alkylsulphonyl, alkylcarbonyl, and alkyl, wherein the alkyl groups have 1-5 carbon atoms,

and which intermediate has either the R or the S configuration,

is prepared by subjecting an optically active cyanohydrin of the general formula

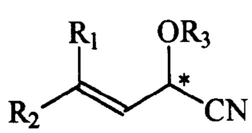

(II)

wherein

$R_1$ and $R_2$ are each individually hydrogen atoms or $(C_1-C_4)$alkyl groups, and

$R_3$ is a hydroxy-protecting group,

to a reduction-transimination-reduction sequence, using R - $NH_2$ as the primary amine,

after which the compound obtained, having the general formula

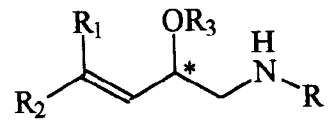

(III)

is converted, if necessary after deprotection of the hydroxy group, to a heterocyclic compound of the general formula

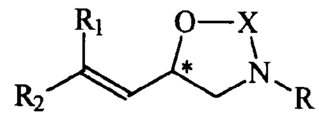

(IV)

which compound is finally subjected to an ozonolysis-reduction sequence.

A main feature of the present invention is the use of easily accessible optically active cyanohydrins of formula II as starting materials. Such optically active cyanohydrins of unsaturated aliphatic aldehydes or ketones, for example the desired enantiomerically pure cyanohydrin of crotonaldehyde, can easily be obtained, e.g. as described by Zandbergen et al. in Synth. Commun. 1991, 21, 1387-91, or alternatively, according to an economically more attractive production process, as described in EP-A-0547655. It is another feature of the method of the present invention, that in the last reaction step, i.e. the ozonolysis-reduction sequence, the chirality remains conserved, or, in other words, the sterical configuration does not change. This is indeed unexpected, because the intermediately formed aldehyde or ketone might easily undergo racemisation via its enol structure. Therefore, the present invention offers a stereoselective synthetic route to heterocyclic intermediates of the above formula I structure, which route is indeed unprecedented.

Examples of suitable heteroaryl groups for the symbol R in the above formula I compounds are furanyl, thienyl, benzofuranyl and benzodioxolyl. Examples of suitable hydroxy-protecting groups $R_3$ are: (trihydrocarbyl)silyl, (dihydrocarbyl)(hydrocarbyloxy)silyl, $(C_4-C_{10})$tert.-alkyl, phenoxy[di$(C_1-C_4)$alkyl)]methyl, $(C_1-C_4)$alkoxy-[di$(C_1-C_4)$alkyl]methyl, and (thio)acetal-constituting groups such as di- and tetrahydropyran-2-yl and di- and tetrahydrofur-2-yl.

The term hydrocarbyl includes $(C_1-C_8)$alkyl and phenyl.

The term (ar)alkylidene includes straight and branched alkylidene and phenylalkylidene such as benzylidene and phenylethylidene.

The optional deprotection of the hydroxy group can be performed in a manner known in the art. In the case of acid-labile protecting groups, such as the above-defined silyl protecting groups, alkoxy(dialkyl)methyl groups or (subst.)phenoxy(dialkyl)methyl groups, this can be accomplished, for example, by a treatment with an aqueous acid.

The above-mentioned reduction-transimination-reduction sequence can be performed in a corresponding manner as described by Zandbergen et al.: Tetrahedron 1992, 48 (19), 3977-82; EP-A-0554933. For that purpose the optically active cyanohydrin of formula II is partly reduced with a reducing agent of the formula $(R_4)_p$ - A - H, wherein A represents a hydrogen atom or a metal atom selected from the group of alkali, earth alkali or early transition metal atoms, R is a $(C_1-C_6)$alkyl, $(C_2-C_6)$alkoxyalkyl or $(C_1-C_6)$alkoxy group, and p is, dependent on the valence of A, 0 - 2; followed by a transimination reaction of the intermediate imino-compound, using R - $NH_2$ as the primary amine, and a final reduction.

The above partial reduction can be carried out with organometallic reagents containing an active hydrogen atom or with hydrogen and a selected metallic catalyst. Suitable organometallic reagents are e.g. aluminium or boron hydrides, such as diisobutylaluminium hydride (DIBAL) or sodium di(2-methoxyethoxy)aluminium hydride. These reductions are preferably carried out in apolar solvents such as ethers, tert. amines, alkanes or aromatic solvents, or mixtures thereof.

The above transimination reaction is preferably carried out after the addition of a proton donating agent such as an alcohol (e.g. methanol) or an ammonium salt (e.g. ammonium bromide). The subsequent addition of the primary amine of formula R - $NH_2$ gives rise to a fast and irreversible transimination reaction under the liberation of $NH_3$, producing a secondary imine.

The final reduction can be established by the usual reducing agents employed for the conversion of imines into secondary amines, e.g. as described by Harada in "The Chemistry of the Carbon-Nitrogen Double Bond". pp. 276-293. Useful examples are (earth)alkali metal aluminiumhydrides and borohydrides, (earth)alkali metals

in protic solvents, and hydrogen gas in the presence of a metal catalyst. Advantageously use is made of reducing agents of the general structure $M_1M_2(Q)_nH_{4-n}$, wherein $M_1$ is a metal from the group IA or IIA of the periodic table of elements, $M_2$ is boron or aluminium, n is an integer having the value 0-3, and Q is an electron-withdrawing substituent, e.g. of the type CN, halogen, alkoxy or dialkylamino. In particular, use can be made of a reagent wherein $M_1$ is an alkali metal, $M_2$ is boron, n is 0 or 1 and Q is CN.

The above-mentioned conversion of the compound of formula III (or of its deprotected form) to a heterocyclic compound of formula IV is explained in more detail hereinafter.

As stated above, the optically active cyanohydrins of formula II are generally easily accessible. If, however, one of the optical isomers is less easily accessible than its optical antipode, this former isomer may be obtained from its optical antipode by an inversion step. Such an inversion of configuration is known in the art, e.g. from a publication by Kano et al. (Tetrahedron Letters 1987, 28 (50), 6331-34), or by a Mitsunobu esterification reaction (Warmerdam et al., Tetrahedron 1993, 49 (5), 1063-70). In the former method (Kano et al.), first a carbobenzyloxy group is introduced on the amino group of the compound of formula III, followed by a ring closure with inversion, using thionylchloride or trifluoromethanesulfonic anhydride as a reagent, to yield the oxazolidinone compound (formula IV; X = CO) of opposite configuration. According to the Mitsunobu esterification reaction, the inversion of configuration is performed with the optically active cyanohydrin of formula II. This cyanohydrin is transformed into a cyanohydrin ester of opposite configuration under so-called Mitsunobu conditions (diethyl azodicarboxylate, triphenylphosphine, carboxylate nucleophile) and subsequently solvolysed, to produce the desired cyanohydrin. The solvolysis is preferably carried out with a sulfonic acid, such as methanesulfonic acid or p-toluenesulfonic acid.

The above-described method of the present invention is preferably carried out via an intermediate oxazolidinone compound as the heterocyclic formula IV compound. Such a oxazolidinone compound of the general formula

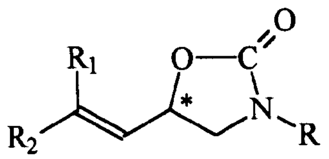

(V)

can easily be prepared by reacting the above compound of formula III, if necessary after deprotection of the hydroxy group, with a suitable carbonylating agent. The easy accessibility of oxazolidinone compounds favours a reaction route via these heterocyclic intermediates.

The above-described ozonolysis-reduction sequence can successfully be carried out by passing ozone through a solution of the above-defined compound IV or V in an inert organic solvent, followed by a reduction of the formed ozonide with a suitable reducing agent. Suitable reducing agents for the ozonide are: a transition metal combined with a proton source, such as Zn in acetic acid; a metal hydride, such as sodiumborohydride; hydrogen under the influence of a hydrogenation catalyst, such as palladium (on a carrier), platina or Raney nickel; or a suitable organic phosphine, phosphite or sulphite, such as triphenylphosphine, trimethylphosphite or dimethylsulphite, if desired followed by a final hydrogenation step.

Suitable organic solvents are chlorinated hydrocarbons, such as dichloromethane, or alcohols, such as e.g. ethanol and butanol.

In general the intermediate heterocyclic compounds of the above formula IV are new. Therefore the present invention also relates to a heterocyclic compound of the general formula

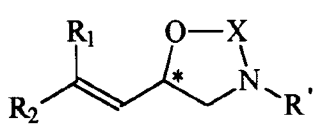

(IV')

wherein

$R_1$ and $R_2$ have the above meanings, and

R' is a straight or branched $(C_1$-$C_{10})$alkyl group, optionally substituted with halogen, hydroxy, $(C_1$-$C_4)$alkoxy or protected hydroxy,

said compound having either the R or the S configuration.

More in particular this new intermediate can be presented by the general formula

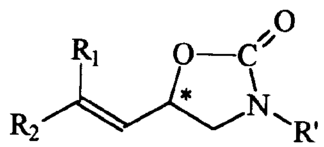

(V')

wherein the symbols have the above meanings,
said compound having either the R or the S configuration.

The present invention further relates to a method of preparing the above new heterocyclic compounds of the general formula IV', by converting a compound of the general formula

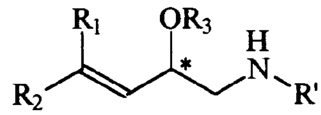

(III')

wherein the symbols have the above meanings and which compound has either the R or the S configuration, if necessary after deprotection of the hydroxy group,
with a (thio)carbonylating agent, with a ($C_1$-$C_{10}$)(ar)alkyl ketone or aldehyde, or a ketal or acetal thereof, or with a dihydrocarbylsilyldihalogenide.

The same method can be used for the preparation of the above-defined heterocyclic compounds of the general formula IV.

For the preparation of new oxazolidinone compounds of the general formula V', as defined above, a compound of formula III' can be converted with a carbonylating agent, if necessary after deprotection of the hydroxy group. Such a carbonylation reaction is preferably carried out in an inert organic solvent such as a chlorinated hydrocarbon, in an inert atmosphere, and at a temperature between -20°C and the boiling point of the solvent, preferably at a slightly reduced temperature such as 0°C. Suitable carbonylating agents are fosgene, diethylcarbonate, ethyl chloroformate and di(heteroaryl)carbonyls such as carbonyldiimidazole.

The present invention finally relates to the use of a heterocyclic compound of the general formula IV', as defined hereinbefore, for the production of an optically active aryloxy-substituted vicinal aminoalcohol of the general formula

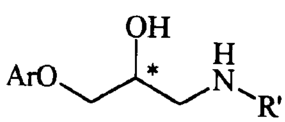

(VI)

wherein

Ar is an optionally substituted aryl or heteroaryl group, and

R' has the above meaning,

by first subjecting said heterocyclic compound to an ozonolysis-reduction sequence, to produce a heterocyclic intermediate of the general formula

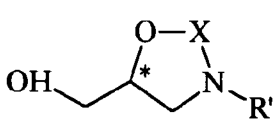

(I')

wherein X and R' have the above meanings, and which compound has either the R or the S configuration, and by then converting the hydroxy group of said heterocyclic intermediate into a suitable leaving group, such as a halogen atom or a sulphonyloxy (e.g. tosyloxy or mesyloxy) group, followed by a reaction with a nucleophilic aryloxy moiety.

The ozonolysis-reduction sequence is described in greater detail hereinbefore. The reaction conditions of the final conversion are wellknown in the art.

Preferred examples of aryloxy-substituted vicinal aminoalcohols, for the production of which the heterocyclic compounds of the above general formula IV' can be used, are compounds of the above general formula VI, wherein R' is an isopropyl, a tert.-butyl or a tert.-pentyl group, and Ar is one of the groups represented by the formulas **7** to **39**. These preferred optically active compounds, which are conveniently accessible by using heterocyclic compound IV' as starting material according to the invention, all belong to the class of β-adrenoceptor antagonists.

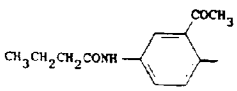
7

8
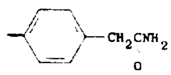

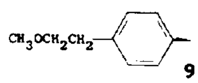
9

10
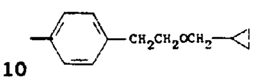

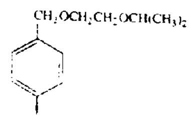
11

12
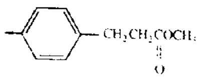

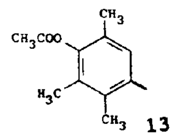
13

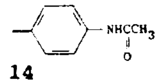
14

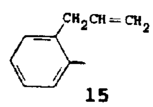
15

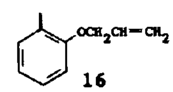
16

17
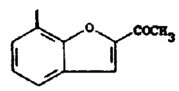

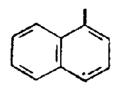
18

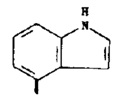
19

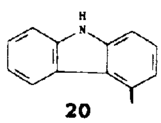
20

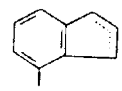
21

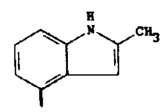
22

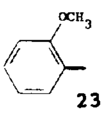
23

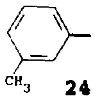
24

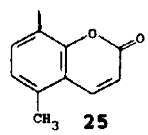
25

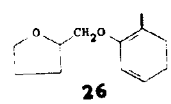
26

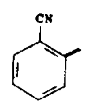
27

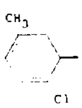
28

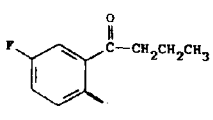
29

30
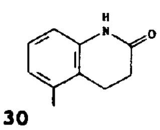

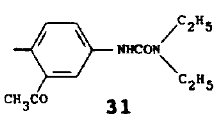
31

32
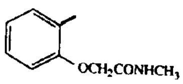

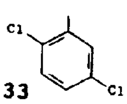
33

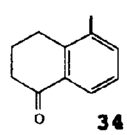
34

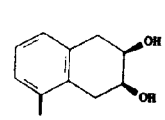
35

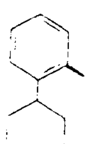
36

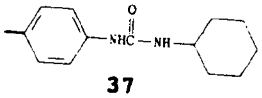
37

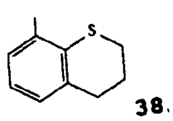
38.

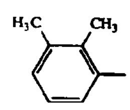
39

The invention will now be described in greater detail with reference to the following specific Examples.

**Reaction route**:

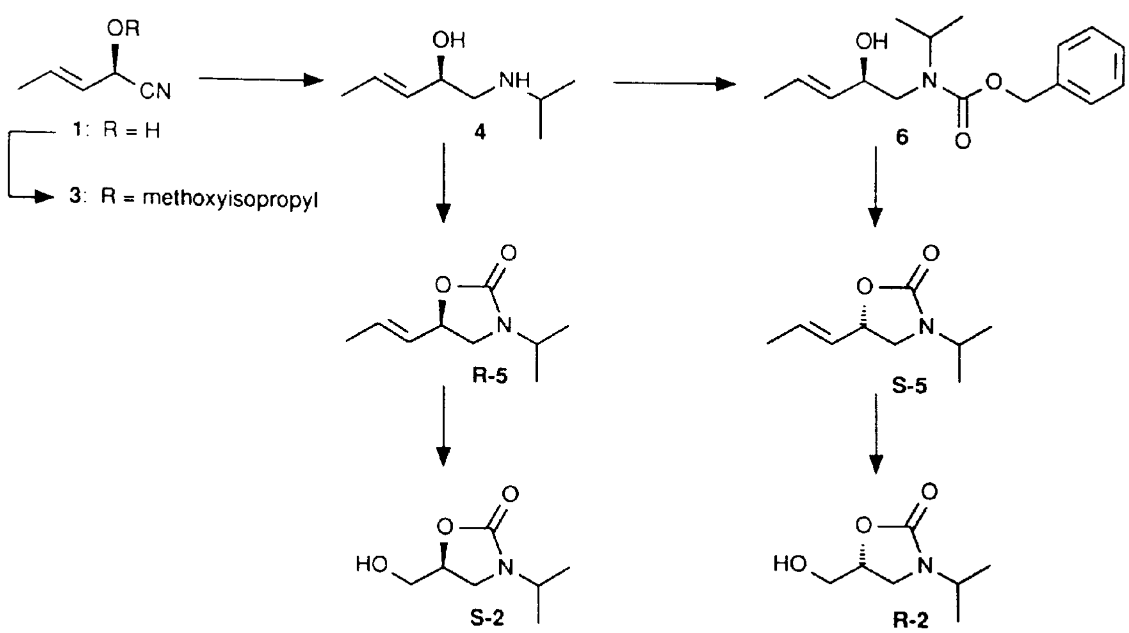

Example I

Preparation of (R)-3-(methylethyl)-5-(1-propenyl)oxazolidinone (**R-5**)

(a). (R)-(2-hydroxy)pent-3-enenitrile (**1**; 9.7 g, 100 mmol),obtained as described in EP-A-0547655, is dissolved in 30 ml 2-methoxypropene. A catalytic amount (13 mg) of $POCl_3$ is added and the mixture is stirred at room temperature for 15 min. Triethylamine (29 mg) is then added, the mixture is taken up in diethylether (100 ml) and washed with saturated brine (2 x 10 ml). The organic layers are dried over $MgSO_4$ and concentrated in vacuo to yield 16.4 g (97%) of (R)-[(2-methoxyisopropyl)oxy]pent-3-enenitrile (3) as an oil.
$^1$H-NMR: δ(ppm) 1.38 and 1.48 (s, 6H, $C(CH_3)_2$); 1.78 (d, 3H, $CHCH_3$); 3.24 (s, 3H, $OCH_3$); 4.91 (d, 1H, CHCN); 5.53 (dd, 1H, $CHCHCH_3$); 6.05 (m, 1H, $CHCH_3$).
$^{13}$C-NMR: δ(ppm) 131.55 (**C**$HCH_3$); 124.30 (**C**$HCHCH_3$); 118.46 (**C**N); 102.11 (**C**$(CH_3)_2$); 59.39 (**C**HCN); 49.35 (O**C**$H_3$); 24.67 and 24.00 (C(**C**$H_3)_2$); 17.32 (CH**C**$H_3$).
$[\alpha]_D^{20}$= +32° (c=1 $CHCl_3$); e.e. 96%.

(b). To a cooled solution (-70°C) of 3.34 g (20 mmol) of **3** in 160 ml of dry diethylether is added 40 ml of a 1M DIBAL solution in cyclohexane (40 mmol). After stirring at -70°C for 3 hours, 4.0 g $NH_4Br$ (40 mmol) in 60 ml dry methanol is added. The cooling bath is removed and 5.9 g isopropylamine (100 mmol) is added. Stirring is continued for 45 minutes in which the temperature is allowed to reach room temperature. The mixture is then cooled with an ice bath and 1.5 g $NaBH_4$ (40 mmol) is added in three portions. The reaction mixture is then stirred for another two hours. 1N HCl solution (250 ml) is added and the layers are separated after extraction. The organic layer is extracted with an additional 50 ml 1N HCl solution. The water layer is made alkaline with 5N NaOH solution until a clear solution appears and extracted with dichloromethane (5 x 50 ml). The combined organic layers are dried over $K_2CO_3$ and concentrated in vacuo to yield 2.31 g (81%) of the desired (R)-1-(methylethyl)aminopent-3-en-2-ol (**4**) as an oil.
$^1$H-NMR: δ(ppm) 1.06 (d, 6H, $CH(CH_3)_2$); 1.70 (d, 3H, $CHCH_3$); 2.24 (br., 2H, **OH** and **NH**); 2.49 (dd, ABX, 1H, $CH_2N$, $J_{AB}$ = 11.99 Hz, $J_{AX}$ = 8.56 Hz); 2.74 (dd, ABX, 1H, $CH_2N$, $J_{AB}$ = 11.99 Hz, $J_{BX}$ = 3.85 Hz); 2.80 (m, 1H, $CH(CH_3)_2$); 4.04 (m, 1H, CHOH); 5.46 (m, 1H, $CHCHCH_3$); 5.73 (m, 1H, $CHCH_3$).
$^{13}$C-NMR: δ(PPM) 132.16 (**C**$HCH_3$); 126.82 (**C**$HCHCH_3$); 70.43 (**C**HOH); 52.68 (**C**$H_2$N); 48.39 (**C**$H(CH_3)_2$); 22.60 (CH(**C**$H_3)_2$); 17.49 (CH**C**$H_3$).

$[\alpha]_D^{20}$ = -35° (c=1 $CH_2Cl_2$); e.e. 96%.

(c). Compound **4** (1.43 g, 10 mmol) is dissolved in 30 ml of dry dichloromethane under a nitrogen atmosphere at 0°C. Carbonyldiimidazole (3.2 g, 20 mmol) is added and the reaction mixture is stirred overnight. The mixture is then acidified (pH = approx. 3) with a 0.5 N HCl solution and the layers are separated after extraction. The water layer is extracted with dichloromethane (4 x 25 ml). The combined organic layers are dried over $MgSO_4$ and concentrated in vacuo, to yield 1.26 g (75%) of the title compound **R-5**.

$^1$H-NMR: δ(ppm) 1.16 (dd, 6H, CH($\mathbf{CH_3}$)$_2$); 1.75 (dd, 3H, CHC$\mathbf{H_3}$); 3.16 (dd, ABX, 1H, C$\mathbf{H_2}$N, $J_{AB}$ = 8.56 Hz, $J_{AX}$ = 7.49 Hz); 3.57 (t, ABX, 1H, C$\mathbf{H_2}$N, $J_{AB}$ = 8.56 Hz, $J_{BX}$ = 8.56 Hz); 4.11 (m, 1H, C**H**($CH_3$)$_2$); 4.85 (q, 1H, C**H**O); 5.54 (m, 1H, C**H**CHCH$_3$); 5.84 (m, 1H, C**H**CH$_3$).

$^{13}$C-NMR: δ(ppm) 156.75 (**C**O); 130.96 (**C**HCH$_3$); 127.49 (**C**HCHCH$_3$); 73.85 (**C**HO); 44.85 (**C**H$_2$N); 44.24 (**C**H(CH$_3$)$_2$); 19.51 and 19.04 (CH(**C**H$_3$)$_2$); 17.23 (CH**C**H$_3$).

$[\alpha]_D^{20}$= + 28° (c=1 $CH_2Cl_2$); e.e. 96%.

Example II

Preparation of (S)-3-(methylethyl)-5-(1-propenyl)oxazolidinone (**S-5**)

(a). Compound **4**, prepared as described in Example I (1.44 g, 10 mmol), is dissolved in 8 ml water at 5°C. Benzyl chloroformate (1.58 g, 11 mmol) is added followed by 1.66 g (12 mmol) of potassium carbonate, and the reaction mixture is stirred for 4 hours. The mixture is then acidified and extracted with dichloromethane (3 x 20 ml). The combined organic layers are washed with saturated brine (10 ml), dried over $MgSO_4$ and concentrated in vacuo. The crude product is purified over silica gel using diethylether : light petroleum 1:1 as eluent. The desired (R)-N-carbobenzyloxy-1-(methylethyl)aminopent-3-en-2-ol (**6**) is obtained in a yield of 2.68 g (96%).

$^1$H-NMR: (at 60°C) δ(ppm) 1.17 (dd, 6H, CH(C**H**$_3$)$_2$); 1.68 (d, 3H, CHC**H**$_3$); 3.16 (dd, ABX, 1H, C**H**$_2$N, $J_{AB}$ = 14.65 Hz, $J_{AX}$ = 3.60 Hz); 3.32 (dd, ABX, 1H, C**H**$_2$N, $J_{AB}$ = 14.65 Hz, $J_{BX}$ = 8.22 Hz); 4.18 (m, 1H, C**H**(CH$_3$)$_2$); 4.23 (m, 1H, C**H**OH); 5.15 (s, 2H, OC**H**$_2$C$_6$H$_5$); 5.49 (m, 1H, C**H**CHCH$_3$); 5.71 (m, 1H, C**H**CH$_3$); 7.34 (m, 5H, arom).

$^{13}$C-NMR: (at 60°C) δ(ppm) 157.30 (**C**O); 136.66 (ipso arom); 131.84 (**C**HCH$_3$); 128.36 (arom); 127.87 (arom); 127.75 (**C**HCHCH$_3$); 127.17 (arom); 72.88 (**C**HOH); 67.19 (O**C**H$_2$C$_6$H$_5$); 50.17 (**C**H$_2$N); 48.88 (**C**H(CH$_3$)$_2$); 20.76 and 20.56 (CH(**C**H$_3$)$_2$); 17.41 (CH**C**H$_3$).

$[\alpha]_D^{20}$ = +13° (c=1 $CH_2Cl_2$); e.e. 96%.

(b). Compound **6** (2.68 g, 9.6 mmol) is dissolved in 12 ml thionyl chloride at 0°C and stirred for 17 hours at room temperature. The reaction mixture is then poored on ice and extracted with dichloromethane (4 x 25 ml). The combined organic layers are dried over $MgSO_4$ and concentrated in vacuo, to yield the title compound as a crude product. Purification over silica using diethylether : light petroleum as the eluent yields 0.82 g (4.8 mmol) of **S-5** as a colourless oil.

The analytical data are in agreement with those for compound **R-5** with $[\alpha]_D^{20}$ = -28° (c=1 $CHCl_3$) for **S-5**; e.e. 96%.

Example III

Preparation of (S)-5-hydroxymethyl-3-(methylethyl)oxazolidinone (**S-2**)

Compound **R-5** (0.85 g, 5 mmol), obtained as described in Example I, is dissolved in 30 ml dichloromethane and 2 ml methanol, and then cooled to -70°C. Ozone is passed through the solution until a blue colour persists. The reaction mixture is stirred for one hour allowing the temperature to reach -30°C, after which 0.18 g (5 mmol) of $NaBH_4$ is added. After stirring for 2 hours at -30°C, 3 ml of a 6N HCl solution is introduced. The water is removed by azeotropic distillation with toluene, and the residue is passed over a silica gel column using dichloromethane as eluent. Concentration in vacuo and subsequent crystallization from diethylether yields 0.64 g (80%) of the title compound **S-2**.

$^1$H-NMR: δ(ppm) 1.18 (dd, 6H, CH(C**H**$_3$)$_2$); 2.38 (br, 1H, O**H**); 3.41 (dd, ABX, 1H, C**H**$_2$N, $J_{AB}$ = 8.57 Hz, $J_{AX}$ = 6.64 Hz); 3.53 (dd, ABX, 1H, $J_{AB}$ = 8.57 Hz, $J_{BX}$ = 8.78 Hz); 3.65 (dd, ABX, 1H, C**H**$_2$OH, $J_{AB}$ = 12.42 Hz, $J_{AX}$ = 4.29 Hz); 3.88 (dd, ABX, 1H, C**H**$_2$OH, $J_{AB}$ = 12.42 Hz, $J_{BX}$ = 3.42 Hz); 4.11 (m, 1H, C**H**(CH$_3$)$_2$); 4.59 (m, 1H, HOCH$_2$C**H**).

$^{13}$C-NMR: δ(ppm) 157.75 (**C**O); 73.41 (HOCH$_2$**C**H); 62.34 (HO**C**H$_2$); 44.36 (**C**H(CH$_3$)$_2$); 40.62 (**C**H$_2$N); 19.30 and 19.07 (CH(**C**H$_3$)$_2$).

$[\alpha]_D^{20}$ = + 53° (c=1 $CHCl_3$); m.p. = 53-55 °C; e.e. ≧ 99%.

In a corresponding manner (R)-5-hydroxymethyl-3-(methylethyl)-oxazolidinone (**R-2**) is prepared from compound **S-5**. The analytical data are identical to those for **S-2** with $[\alpha]_D^{20}$ = -53° (c=1 $CHCl_3$) for **R-2**; e.e. ≧ 99%.

Example IV

Use of (S)-5-hydroxymethyl-3-(methylethyl)oxazolidinone (**S-2**) for the preparation of (S)-propranolol.

(a) The starting compound (**S-2**) is mesylated as follows:
To a solution of 3.3 g (21 mmol) **S-2**, 3.0 g (30 mmol) triethylamine and a catalytic amount of (N,N-dimethylamino)pyridine in 30 ml chloroform is added 2.6 g (23 mmol) methanesulfonyl chloride; the reaction mixture is stirred at 0°C for 2 hours. Then water is added and the mixture is extracted with methylene chloride. After removal of the solvent by evaporation, (S)-5-[(methylsulfonyloxy)methyl]-3-(methylethyl)-oxazolidinone is obtained in a quantitative yield.
(b) Preparation of the naphthyloxy-compound:
To a solution of the product of (a) (4.7 g, 20 mmol) in 35 ml benzene is added 20 g Amberlyst® A26 in the naphtholate form; the suspension is stirred at room temp. for 24 hours. The resin is filtered off, the solvent removed in vacuo, and the residue purified by column chromatography over silica gel with cyclohexane - ethyl acetate (1/1) as the eluent. (S)-5-(naphthyloxymethyl)-3-(methylethyl)oxazolidinone is obtained in a yield of approx. 70%.
(c) Preparation of (S)-propranolol
A solution of the product of (b) (2.88 g, 10 mmol) in 10 ml ethanol is added to a 4N KOH solution; the reaction mixture is refluxed for 24 hours. The reaction mixture is extracted with benzene, and the solvent is removed in vacuo. After acidification with 2N HCl and recrystallization from EtOH-$Et_2O$, optically pure (S)-propanolol.HCl is obtained in a yield of approx. 60%; m.p. 191-193°C; $[\alpha]_D^{20}$ = -25° (c= 1, EtOH); the $^1$H-NMR spectrum is identical with that reported in literature (Kan et al., Agric. Biol. Chem. 49 (1), 1985, 207-210).

## Claims

1. A method of preparing a heterocyclic intermediate for the production of optically active (enantiomerically pure) aryloxy-substituted vicinal aminoalcohols, characterized in that a heterocyclic intermediate of the general formula

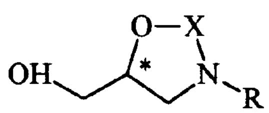

(I)

wherein

X is a carbonyl group, a thiocarbonyl group, a ($C_1$-$C_{10}$)(ar)alkylidene group or a dihydrocarbylsilyl group,

R is a straight or branched ($C_1$-$C_{10}$)alkyl group, optionally substituted with halogen, hydroxy, ($C_1$-$C_4$)alkoxy or protected hydroxy, or a phenyl($C_1$-$C_3$)alkyl or heteroaryl($C_1$-$C_3$)alkyl group, which groups are optionally substituted with 1-3 substituents, selected from the group consisting of hydroxy, ($C_5$-$C_{12}$) cycloalkyl, amino, nitro, halogen, cyano, alkoxy, alkylcarbonyloxy, alkylcarbonylamino, alkylsulphonylamino, alkylsulphonyl, alkylcarbonyl, and alkyl, wherein the alkyl groups have 1-5 carbon atoms,

and which intermediate has either the R or the S configuration,

is prepared by subjecting an optically active cyanohydrin of the general formula

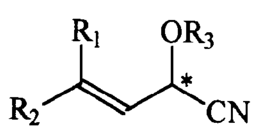

(II)

wherein

$R_1$ and $R_2$ are each individually hydrogen atoms or $(C_1\text{-}C_4)$alkyl groups, and
$R_3$ is a hydroxy-protecting group,

to a reduction-transimination-reduction sequence, using R - $NH_2$ as the primary amine, after which the compound obtained, having the general formula

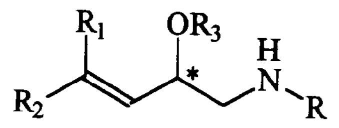

(III)

is converted, if necessary after deprotection of the hydroxy group, to a heterocyclic compound of the general formula

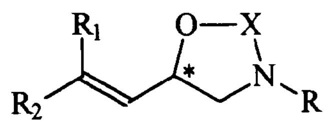

(IV)

which compound is finally subjected to an ozonolysis-reduction sequence.

2. A method as claimed in claim 1, characterized in that a heterocyclic intermediate of the general formula I, wherein X is a carbonyl group and which intermediate has either the R or the S configuration, is prepared by reacting a compound of the general formula III, prepared according to claim 1, with a carbonylating agent, to produce an oxazolidinone compound of the general formula

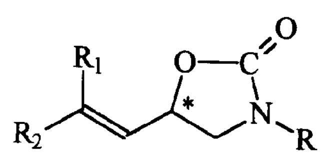

(V)

wherein the symbols have the meanings given in claim 1,
which compound is finally subjected to an ozonolysis-reduction sequence.

3. A method as claimed in claim 1 or 2, wherein the ozonolysis-reduction sequence is carried out by passing ozone through a solution of compound IV or V in an inert organic solvent, followed by a reduction of the formed ozonide with a transition metal combined with a proton source, with a metal hydride, with hydrogen under the influence of a hydrogenation catalyst, or with an organic phosphine, phosphite or sulphite, if necessary followed by a final hydrogenation step.

4. A heterocyclic compound of the general formula

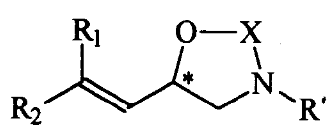

(IV')

wherein

$R_1$ and $R_2$ have the meanings given in claim 1, and
R' is a straight or branched $(C_1\text{-}C_{10})$alkyl group, optionally substituted with halogen, hydroxy, $(C_1\text{-}$

$C_4$)alkoxy or protected hydroxy, said compound having either the R or the S configuration.

**5.** A compound as claimed in claim 4, having the general formula

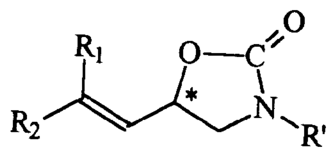

(V')

wherein

$R_1$ and $R_2$ have the meanings given in claim 1, and

R' has the meaning given in claim 4,

said compound having either the R or the S configuration.

**6.** A method of preparing a heterocyclic compound of the general formula IV', presented in claim 4, characterized in that a compound of the general formula

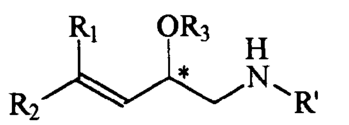

(III')

wherein

$R_1$ and $R_2$ have the meanings given in claim 1, and

R' has the meaning given in claim 4,

said compound having either the R or the S configuration, is converted, if necessary after deprotection of the hydroxy group, with a (thio)carbonylating agent, with a ($C_1$-$C_{10}$)(ar)alkyl ketone or aldehyde, or a ketal or acetal thereof, or with a dihydrocarbylsilyldihalogenide.

**7.** A method as claimed in claim 6 of preparing a compound of the general formula V', presented in claim 5, characterized in that a compound of the general formula III', as defined in claim 6, is converted, if necessary after deprotection of the hydroxy group, with a carbonylating agent, preferably selected from fosgene, diethylcarbonate, ethyl chloroformate and di(heteroaryl)carbonyls.

**8.** Use of a heterocyclic compound of the general formula IV', as defined in claim 4, for the production of an optically active aryloxy-substituted vicinal aminoalcohol of the general formula

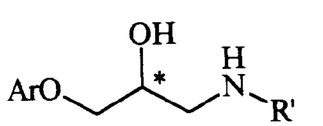

(VI)

wherein

Ar is an optionally substituted aryl or heteroaryl group, and

R' has the meaning given in claim 4,

by first subjecting said heterocyclic compound to an ozonolysis-reduction sequence, to produce a heterocyclic intermediate of the general formula

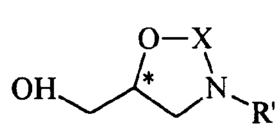

(I')

wherein

X has the meaning given in claim 1, and

R' has the meaning given in claim 4,

said compound having either the R or the S configuration,

and by then converting the hydroxy group of said heterocyclic intermediate into a suitable leaving group, followed by a reaction with a nucleophilic aryloxy moiety.

9. Use, as defined in claim 8, of a heterocyclic compound of the general formula IV', for the production of an optically active aryloxy-substituted vicinal aminoalcohol of the general formula VI, wherein R' is an isopropyl group, a tert.-butyl or a tert.-pentyl group, and Ar is one of the groups represented by the formulas **7** to **39**:

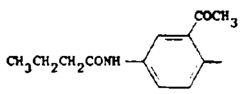
7
8
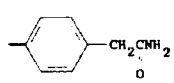
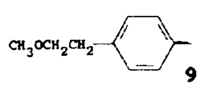
9
10
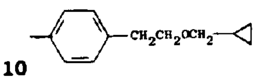

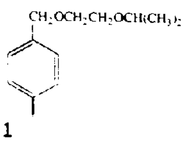
11
12
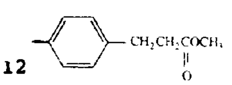
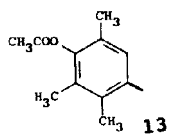
13
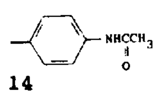
14

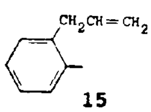
15
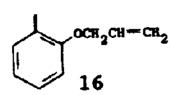
16
17
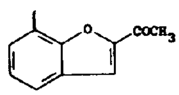
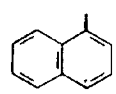
18

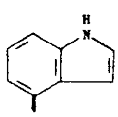
19
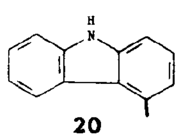
20
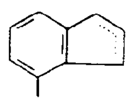
21
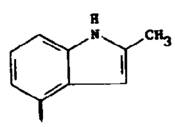
22

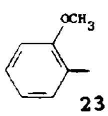
23
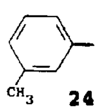
24
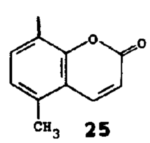
25
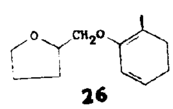
26
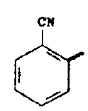
27

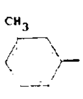
28
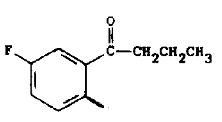
29
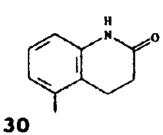
30
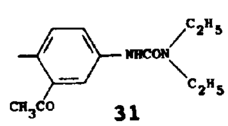
31

32
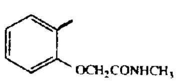
33
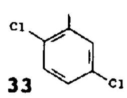
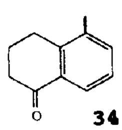
34
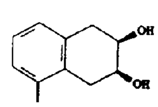
35

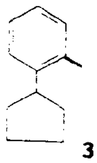
36

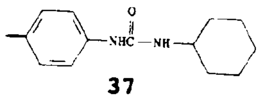
37

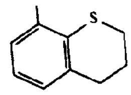
38

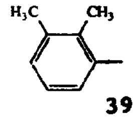
39

European Patent Office

# EUROPEAN SEARCH REPORT

Application Number
EP 94 20 2887

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | SYNTHETIC COMMUNICATIONS, vol.19, no.19, 1989, NEW YORK pages 3313 - 3321 D.SUBHAS BOSE ET AL 'synthesis of (R)-(-)-gamma-amino-beta-hydroxy butyric acid(gabob)' *page 3315,Scheme 2,compound 9,page 3319* | 1,4-6 | C07D263/24<br>C07D263/16 |
| D,A | JOURNAL OF ORGANIC CHEMISTRY., vol.53, no.23, 11 November 1988, EASTON US pages 5531 - 5534 DANIELE BIANCHI ET AL 'Anhydrides as acylating agents in lipase-catalysed stereoselective esterification of racemic alcohols' * the whole document * | 1 | |
| D,A | EP-A-0 554 933 (DUPHAR INTERNATIONAL RESEARCH B. V) * claims * | 1 | |
| A | EP-A-0 339 006 (ASTRA PHARMACEUTICAL PRODUCTION AB) * claims * | 1,8,9 | TECHNICAL FIELDS SEARCHED (Int.Cl.6)<br>C07D |
| A | CHEMICAL AND PHARMACEUTICAL BULLETIN., vol.29, no.12, December 1981, TOKYO JP pages 3593 - 3600 YOSHISUKE TSUDA ET AL 'Practical syntheses of [R]- and [S]-1-alkylamino-3-aryloxy-2-propanols from a single carbohydrate precursor' * the whole document * | 1,8,9 | |

The present search report has been drawn up for all claims

| Place of search | Date of completion of the search | Examiner |
|---|---|---|
| THE HAGUE | 14 December 1994 | Henry, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)